# EUROPÄISCHE PATENTANMELDUNG

(11) **EP 0 646 346 A2**
(43) Veröffentlichungstag der Anmeldung: **05.04.1995**
(21) Anmeldenummer: 94110770.8
(22) Anmeldetag: 11.07.1994
(51) Int. Cl.: A61B 5/083, A61B 8/00

(54) **Vorrichtung zur Messung von Atemgasparametern**

(30) Priorität: 30.09.1993 DE 4333410; 21.03.1994 DE 4409589
(71) Anmelder: NDD Medizintechnik GmbH, D-97003 Würzburg (DE)
(72) Erfinder: Harnoncourt, Karl, Prof. Dr., A-8010 Graz (AT); Guggenbühl, Walter, Prof. Dr., CH-8712 Stäfa (CH); Schlegelmilch, Rolf M., D-97074 Würzburg (DE); Buess, Christian, CH-8003 Zürich (CH)
(74) Vertreter: Laufhütte, Dieter, Dr.-Ing.

(57) **Zusammenfassung**

Die Erfindung betrifft eine Vorrichtung zur Messung von Atemgasparametern mit einem Atemrohr, Ultraschallsensoren und einer in einem separaten Gehäuse angeordneten Vorverstärkerelektonik. Um gefährliche Kreuzinfektionen zwischen den Patienten bei Mehrfachverwendung der Meßvorrichtung zu verhindern, sind die Ultraschallsensoren in dem Atemrohr fest integriert, so daß sie mit diesem ein einstückiges Teil bilden, wobei zumindest das Atemrohr mit den integrierten Ultraschallsensoren als austauschbares Teil zur einmaligen Verwendung ausgebildet ist.

## Beschreibung

Die Erfindung betrifft eine Vorrichtung zur Messung von Atemgasparametern mit einem Atemrohr, Ultraschallsensoren und einer in einem separaten Gehäuse angeordneten Vorverstärkerelektronik.

Üblicherweise werden heute vor allem in der Intensivpflege und der Anästhesie, aber auch in der allgemeinen medizinischen Diagnostik, diskrete Sensoren zur Messung der Strömung und der Zusammensetzung von Atemgasen und anderer Größen, wie beispielsweise des Druckes, verwendet. Die Atemgasströmung wird nach unterschiedlichen Prinzipien, zum Beispiel mittels Pneumotachographie oder mittels der Anemometrie am Tubus oder im Beatmungsgerät erfaßt. Die Gaskonzentrationen des Atemgases werden davon unabhängig vorwiegend im Neben- und teilweise Hauptstrom nach unterschiedlichen Prinzipien, zum Beispiel mittels Infrarotspektroskopie, Paramagnetismus oder Massenspektrometrie bestimmt. Der Atemwegsdruck wird wiederum separat am Mund oder im Beatmungsgerät gemessen. Durch die unterschiedlichen Meßanordnungen entstehen zeitliche Verzögerungen zwischen den einzelnen Meßsignalen. Meistens werden die Signale nicht direkt am Mund gemessen, sonderen an anderen Orten, wodurch eine zeitliche Zuordnung zur Atmung des Patienten erschwert wird. Bei der Gasbestimmung im Nebenstrom treten zudem Transienten auf, die zu Fehlern in der Meßgenauigkeit führen. Außerdem sind die Verfahren häufig in Kombination aufwendig und teuer.

Diese Meßvorrichtungen werden im Krankenhauseinsatz täglich bei einer Vielzahl von Patienten eingesetzt. Hierbei besteht die Gefahr von gefährlichen Kreuzinfektionen zwischen den Patienten.

Aufgabe der Erfindung ist es daher, eine Vorrichtung zur Messung von Atemgasparametern nach dem Oberbegriff des Hauptanspruchs an die Hand zu geben, bei dem die Atemgasparameter gleichzeitig erfaßt werden können.

Diese Aufgabe wurde ausgehend von einer gattungsgemäßen Meßvorrichtung erfindungsgemäß durch die kennzeichnenden Merkmale des Hauptanspruchs gelöst. Demnach sind bei einer Meßvorrichtung für Atemgasparameter mit einem Atemrohr, Ultraschallsensoren und einer in einem separaten Gehäuse angeordneten Vorverstärkerelektronik die Ultraschallsensoren in dem Atemrohr fest integriert, so daß sie mit diesem ein einstückiges Teil bilden. Dabei können die Ultraschallsensoren in entsprechende Ansätze des Atemrohr eingegossen sein. Das Atemrohr bildet mit den integrierten Ultraschallsensoren ein austauschbares Teil zur einmaligen Verwendung, das vor dem Mund des Patienten an einer Maske oder einem Tubus aufsteckbar ist. Das Atemrohr kann für besondere Anwendungen auch gassterilisiert und wiederverwendet werden.

Im Atemrohr kann zusätzlich ein Drucksensor einstückig integriert, vorzugsweise eingegossen, sein.

Das entsprechende Elektronikmodul ist dann so auszulegen, daß der aufgenommene Druckwert ebenfalls verarbeitet wird.

Im Atemrohr kann weiterhin zusätzlich eine Infrarotlichtquelle und gegenüberliegend ein optischer Sensor fest integriert sein. Mittels dieser Sensorik lassen sich nach dem Infrarotabsorptionsverfahren C0₂, N₂0 und andere Anästhesiegase bestimmen. Die Infrarotlichtquelle kann auch neben dem optischen Sensor angeordnet sein, wenn die dem Sensor gegenüberliegende Wand des Atemrohrs verspiegelt und so ausgeformt ist, daß das von der Lichtquelle ausgesandte Licht dort reflektiert wird und vom Aufnehmer empfangen werden kann. Bei dieser Ausführungsform kann auch der optische Sensor im Elektronikgehäuse fest installiert werden. Das ermöglicht eine besondere kompakte Bauform.

Das Atemrohr kann vorteilhaft aus einem lichtdurchlässigen Material, vorzugsweise einem lichtdurchlässigen, medizinischen Polymer, gefertigt sein. In diesem Fall kann die Lichtquelle und der optische Sensor in die Wandung des Atemrohres eingegossen sein, da die Strahlung durch die Wandung durchtreten kann. Sowohl die Lichtquelle wie auch der Sensor werden ebenfalls mit der Elektronik verbunden, so daß sie einerseits mit Strom versorgt werden können, und daß andererseits die Signale zur Weiterverarbeitung abgegeben werden können.

Besonders vorteilhaft ist es, wenn die Infrarotlichtquelle und der zugehörige optische Sensor derart neben dem Ultraschallsensor quer zum Verlauf des Atemrohres eingebaut sind, daß sich die Verbindungslinien zwischen den Ultraschallsensoren und zwischen der Infrarotlichtquelle und dem zugehörigen optischen Sensor kreuzen. Hierdurch ist eine besonders kompakte Bauweise mit geringem Totraum ermöglicht.

Die Infrarotlichtquelle und der zugehörige optische Sensor können auch über einen aufsteckbaren Halter auf das Atemrohr aufsetzbar und mit diesem verbindbar sein. Dadurch können die Infrarotlichtquelle und der zugehörige optische Sensor mehrfach eingesetzt werden, wenn das Atemrohr ausgetauscht wird. Weder die Infrarotlichtquelle noch der optische Sensor kommen mit dem durch das Atemrohr strömenden Atemgas in Berührung.

Im Elektronikgehäuse kann eine Lichtquelle installiert sein, die geeignetes Licht aussendet, welches die Wandung des Atemrohres durchdringt und auf der Innenseite des Atemrohres auf einen dort aufgetragenen, geeigneten, fluoreszierenden Farbstoff auftrifft. Neben der Lichtquelle kann im Elektronikgehäuse ein optischer Aufnehmer angeordnet sein, der nach dem Lumineszenz- bzw. Fluoreszenz-Prinzip die Sauerstoffkonzentration bestimmt. Dabei kann die Wandung des Atemrohres in geeigneter Weise ausgeformt werden, um eine verbesserte Lichtleitung zu gewährleisten.

Gemäß einer Ausführungsform der Erfindung ist das Gehäuse für die Vorverstärkerelektronik an das Atemrohr anflanschbar, wobei die Übertragung der Stromversorgung und der Signale von dem Elektronikgehäuse zu den im Atemrohr integrierten Sensoren und umgekehrt mittels einer kodierten Steckerplatte erfolgt. Das angeflanschte Elektronikgehäuse kommt mit dem Atemstrom des Patienten nicht in Berührung und kann daher wiederverwendet werden. Im Gehäuse für die Vorverstärkerelektronik können weitere Anschlüsse für Datenleitungen vorhanden sein, beispielsweise für die Daten, die aus einem separat angeschlossenen 0₂-Analysator stammen. Beim Vorhandensein eines 0₂-Analysators mit Signalausgang kann das Signal der 0₂-Konzentration der Elektronik des Atemrohres zugeführt werden. Über Molmasse und 0₂-Konzentration läßt sich dann die C0₂-Konzentration genau bestimmen.

Gemäß einer alternativen Ausführungsform kann die Vorverstärkerelektronik auch unmittelbar in den Boden des Atemrohres eingegossen sein.

Schließlich kann das Atemrohr insgesamt so dimensioniert werden, daß es an die bei Kleinkindern verwendeten Anschlüsse von Maske und Beatmungstubus paßt. Somit kann das Atemrohr auch in der Pädiatrie und Neonatologie eingesetzt werden.

Die Erfindung besteht also aus einem Atemrohr zum einmaligen Gebrauch oder nach Gassterilisation mehrmaligen Gebrauch, das direkt vor dem Mund des Patienten an einer Maske oder am Tubus aufgesteckt wird. Das Atemrohr ermöglicht die Messung der Strömung der Atemgase und der Molmasse, einer oder mehrerer Gaskonzentrationen und des proximalen Atemwegsdruckes. Die Atmung oder Beatmung des Patienten wird durch die Meßanordnung nicht behindert oder gestört, da die Messung im freien Rohrquerschnitt erfolgt. Teile der Sensorik, vor allem die Ultraschallsensoren und der Druckwandler, sind direkt in das Atemrohr miteinbezogen. Andere Teile der Sensorik, die als optische Aufnehmer ausgebildet sind, sind ebenfalls in das Atemrohr einbezogen oder werden an diese angeflanscht.

Aus den mit der erfindungsgemäßen Vorrichtung gemessenen Primärparametern können abgeleitete Größen, wie beispielsweise die Sauerstoffaufnahme, die Co₂-Abgabe, der respiratorische Quotient, das Atemzugvolumen, das Atemminutenvolumen, die Atemarbeit und andere errechnet werden.

Weitere Einzelheiten und Vorteile der Erfindung werden anhand eines in der Zeichnung dargestellten Ausführungsbeispieles näher erläutert.

Es zeigen:

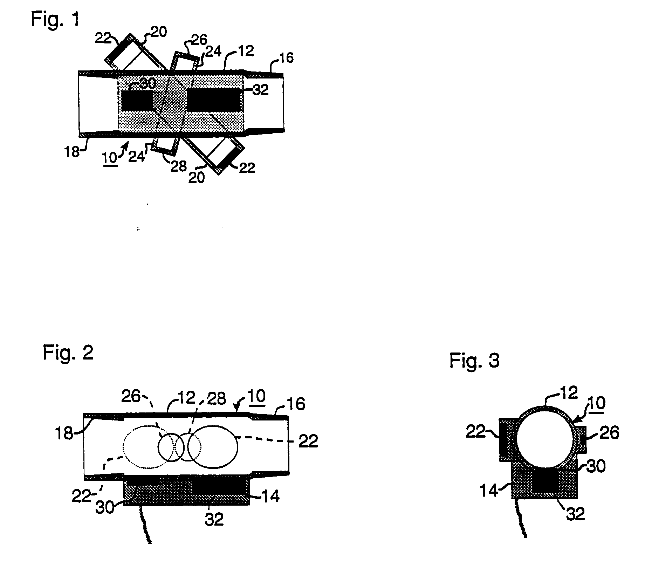

die Figuren 1 bis 3:
drei verschiedene Schnittdarstellungen eines Atemrohres gemäß einer Ausführungsform der vorliegenden Erfindung.

Die Meßvorrichtung 10 besteht im wesentlichen aus einem Atemrohr 12, aus einem lichtdurchlässigen, medizinischen Polymer. Am Atemrohr 12 sind Normanschlüsse 16 und 18 angeformt, die einerseits ein einfaches Anstecken an einer Maske oder an einem Tubus und andererseits ein einfaches Anstecken an eine Schlauchverbindung ermöglichen. Das Atemrohr 12 ist auf ein Gehäuse 14 aufsteckbar, das eine Vorverstärkerelektronik (hier nicht dargestellt) beinhaltet. Beim Aufstecken des Atemrohres 12 auf das Gehäuse der Vorverstärkerelektronik 14 werden über eine kodierte Steckerplatte notwendige Stromversorgungsleitungen und Meßdatenleitungen miteinander verbunden.

Wie insbesondere aus der Figur 1 ersichtlich, sind schräg zur Mittelachse des Atemrohres und sich gegenüberliegend rohrförmige Ansätze 20 am Atemrohr 12 angeformt, die die Meßstrecke für an den Enden der rohrförmigen Anformungen 20 angeordnete Ultraschallsensoren 22 bilden. Die Ultraschallsensoren 22 sind somit in das Atemrohr 12 eingegossen. Sie werden mit diesem zusammen ausgetauscht. Ebenfalls rohrförmige Anformungen 24, die schräg zur Mittelachse des Atemrohres 12 und sich gegenüberliegend angeordnet sind, bilden die Meßstrecke für eine Infrarotlichtquelle 26 und einen zugehörigen optische Sensor 28. Die Meßstrecken der Ultraschallsensoren 22 und der Infrarotlichtquelle 26 sowie des optischen Sensors 28 kreuzen sich, wie in Figur 1 dargestellt. Durch diese Anordnung der Sensoren ist eine besonders kompakte Bauweise möglich.

In der Wandung des Atemrohres 12 ist zusätzlich ein Druckwandler 30 integriert. Weiterhin ist im Gehäuse eine Lichtquelle 32 angeordnet. Gegenüberliegend zu der Lichtquelle 32 ist eine fluoreszierende Schicht in hier nicht dargestellter Art und Weise innen im Atemrohr aufgebracht.

Die mitttels dieser Meßvorrichtung aufgenommenen Signale werden gleichzeitig und ohne zeitlichen Verzug im Hauptstrom erfaßt. Dies ermöglicht eine besonders einfache atemsynchrone Auswertung von Ventilation und Gasaustausch des Patienten. Insbesondere lassen sich Atemvolumina, Atemfrequenz, Sauerstoffaufnahme, C0₂-Abgabe sowie die Konzentrationen von Anästhesiegasen bestimmen. Weiterhin kann mittels Auswaschverfahren die funktionelle Reservekapazität FRC des Patienten ermittelt werden. Die gewonnenen Meßwerte und die abgeleiteten Rechenwerte sind von großer Genauigkeit und bei weit geringerem technischem Aufwand als bei den bisher genannten Meßvorrichtungen. Der Totraum, der bei den vorgenannten Messungen eine wichtige Rolle spielt, ist minimiert.

Die Vorrichtung wird nach der Benutzung durch einen Patienten entsorgt, so daß die gefährliche Kreuzinfektion zwischen den Patienten wirksam verhindert werden kann. Für besondere Anwendungen läßt sich das Atemrohr auch gassterilisieren.

## Patentansprüche

1. Vorrichtung zur Messung von Atemgasparametern mit einem Atemrohr, Ultraschallsensoren und einer in einem separaten Gehäuse angeordneten Vorverstärkerelektronik, dadurch gekennzeichnet, daß die Ultraschallsensoren in dem Atemrohr fest integriert sind, so daß sie mit diesem ein einstückiges Teil bilden, und daß zumindest das Atemrohr mit den integrierten Ultraschallsensoren als austauschbares Teil zur einmaligen Verwendung ausgebildet ist.

2. Vorrichtung nach Anspruch 1, dadurch gekennzeichnet, daß im Atemrohr zusätzlich ein Drucksensor einstückig integriert, vorzugsweise eingegossen, ist.

3. Vorrichtung nach Anspruch 1 oder Anspruch 2, dadurch gekennzeichnet, daß im Atemrohr zusätzlich eine Infrarotlichtquelle und gegenüberliegend ein optischer Sensor fest integriert sind.

4. Vorrichtung nach einem der Ansprüche 1 bis 3, dadurch gekennzeichnet, daß das Atemrohr aus einem lichtdurchlässigen Material, vorzugsweise einem lichtdurchlässigen, medizinischen Polymer, gefertigt ist.

5. Vorrichtung nach Anspruch 3 und Anspruch 4, dadurch gekennzeichnet, daß die Infrarotlichtquelle und der optische Sensor in die Wandung des Atemrohres eingegossen sind.

6. Vorrichtung nach einem der Ansprüche 3 bis 5, dadurch gekennzeichnet, daß die Infrarotlichtquelle und der zugehörige optische Sensor derart neben den Ultraschallsensoren quer zum Verlauf des Atemrohres eingebaut sind, daß sich die Verbindungslinien zwischen den Ultraschallsensoren und zwischen der Infrarotlichtquelle und dem zugehörigen optischen Sensor kreuzen.

7. Vorrichtung nach Anspruch 3, Anspruch 4 oder Anspruch 6, dadurch gekennzeichnet, daß die Infrarotlichtquelle und der zugehörige optische Sensor über einen aufsteckbaren Halter auf das Atemrohr aufbringbar sind.

8. Vorrichtung nach einem der vorangehenden Ansprüche, dadurch gekennzeichnet, daß die Infrarotlichtquelle und der zugehörige optische Sensor nebeneinander angeordnet sind, daß die dem Sensor gegenüberliegende Wand des Atemrohrs verspiegelt und so ausgeformt ist, daß das von der Lichtquelle ausgesandte Licht reflektiert wird und vom Sensor empfangen wird.

9. Vorrichtung nach einem der Ansprüche 1 bis 8, dadurch gekennzeichnet, daß im Elektronikgehäuse eine Lichtquelle installiert ist, die geeignetes Licht derart aussendet, daß dieses die Wandung des Atemrohres durchdringt und auf der Innenseite des Atemrohres auf einen dort aufgetragenen, geeigneten fluoreszierenden Farbstoff auftrifft, daß neben der Lichtquelle im Elektronikgehäuse ein optischer Aufnehmer angeordnet ist, der nach dem Lumineszenz- bzw. Fluoreszenz-Prinzip die Sauerstoffkonzentration bestimmt.

10. Vorrichtung nach einem der Ansprüche 1 bis 9, dadurch gekennzeichnet, daß das Gehäuse für die Vorverstärkerelektronik an das Atemrohr anflanschbar ist, wobei die Übertragung der Stromversorgung und der Signale von dem Elektronikgehäuse zu den im Atemrohr integrierten Sensoren und umgekehrt mittels einer kodierten Steckerplatte erfolgt.

11. Vorrichtung nach einem der Ansprüche 1 bis 10 dadurch gekennzeichnet, daß im Gehäuse für die Vorverstärkerelektronik weitere Anschlüsse für Datenleitungen vorhanden sind, beispielsweise für die Daten, die aus einem separat angeschlossenen 0₂-Analysator stammen.

12. Vorrichtung nach einem der Ansprüche 1 bis 8 oder 10, dadurch gekennzeichnet, daß die Vorverstärkerelektronik unmittelbar in den Boden des Atemrohres eingegossen ist.
